# EUROPEAN PATENT APPLICATION

(11) **EP 2 315 465 A1**
(43) Date of publication of application: **27.04.2011**
(21) Application number: 09013206.9
(22) Date of filing: 20.10.2009
(51) Int. Cl.: H04W 12/12, H04L 29/06, H04W 12/06

(54) **Method for secure communication between devices**

(71) Applicant: ETH Zurich, 8092 Zürich (CH)
(72) Inventor: Castelluccia, Claude, 38190 Bernin (FR); Rasmussen, Kasper Bonne, 8003 Zürich (CH); Capkun, Srdjan, 8049 Zürich (CH)

(57) **Abstract**

A method for communicating between a first device (1) and a second device (2), comprises the steps of
■ the first and second device (1, 2) communicating by exchanging messages that are based on signals that are transmitted through a first communication channel (31) and/or through a second communication channel (32), wherein the first and second communication channel (31, 32) have different signal propagation velocities;
■ at least one of the first and second device (1; 2) computing the distance to the other device (2; 1) based on communication signal delays caused by the signal propagation velocities;
**characterised in that** the method comprises the further steps of
■ controlling access of the second device to the first device depending on the computed distance.

## Description

### FIELD OF THE INVENTION

The invention relates to the field of wireless communication networks, in particular to authentication and access control for devices controlled by wireless communication. It relates to a method for secure communication between two devices, and further relates to a device, in particular an implantable medical device, according to the preamble of the corresponding independent claims.

### BACKGROUND OF THE INVENTION

The paper "Integrity Regions: Authentication Through Presence in Wireless Networks" by Srdjan 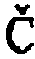 apkun and Mario 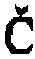 agalj, WiSe'06, September 29, 2006, Los Angeles, presents a security protocol for message authentication in wireless networks without the use of pre-authenticated or pre-established keys. The proximity of a communication partner is verified through time-of-arrival ranging. It is assumed that the two communicating entities involved trust each other. The purpose of the security protocol is to be secure with regard to a man-in-the-middle attack. The communication partners exchange a series of messages, some of them through a wireless RF channel and some through an ultrasound channel. From time delay measurements, the distance between the communicating entities is calculated. Between the message exchanges, cryptographically relevant and computationally expensive calculations take place. After the message exchange, one of the communication partners verifies that (i) the calculated distance is within a certain limit and that (ii) no other device is closer than the calculated distance. The second verification step must be performed visually by a user.

Since the protocol assumes that the communicating entities trust each other, there is no mechanism to prevent that one of the communication partners assumes a false identity.

### DESCRIPTION OF THE INVENTION

It is therefore an object of the invention to create a method of the type mentioned initially, for secure communication between two devices, which overcomes the disadvantages mentioned above. A further object of the invention is to create a device, in particular an implantable medical device, that is able to communicate with a second device such as a reader or control device, which allows for the authentication of the second device and of messages sent by the second device.

These objects are achieved by a method for secure communication between two devices, and are achieved by a device, in particular an implantable medical device, according to the corresponding independent claims.

The method for communicating between a first device and a second device, comprises the steps of
■ the first and second device communicating by exchanging messages that are based on signals that are transmitted through a first communication channel and/or through a second communication channel, wherein the first and second communication channel have different signal propagation velocities;
■ at least one of the first and second device computing the distance to the other device based on communication signal delays caused by the difference in signal propagation velocities;
   characterised in that the method comprises the further steps of
■ controlling access of the second device to the first device depending on the computed distance.

"Access to the first device" means the ability to issue control commands to the first device and/or read data stored in the second device. In a preferred embodiment of the invention, the first device is an implantable medical device (IMD) and the second device is a device for reading data from the IMD and optionally also setting parameters in the IMD or otherwise controlling the IMD, henceforth also called "reader".

The invention allows preventing, on the one hand, that hostile devices, impersonating a reader, can read data from and/or take control of an IMD. On the other hand, it allows preventing devices that impersonate an IMD from fooling a reader, thereby extracting information from the reader or preventing access to the desired IMD. In other words, the invention allows to establish trust as a basis for communication between the two devices.

In a preferred embodiment of the invention, the first and second device, by exchanging the messages, establish a shared secret key. This is preferably done by using a Diffie-Hellman (DH) key establishment protocol, but can be done, in principle with any protocol that establishes a confidential channel over public communication media. However, preferably, energy-intensive operations such as computationally expensive steps are deferred until proximity is verified. "Energy-intensive" means, for example, that in a microprocessor these steps use more than 20 or 50 times the power than the preceding manipulations during proximity verification (e.g., for fetching the bits of the nonce and delivering them to the transmitter), excluding the power required to drive the transmitter. Preferably, the power for the proximity verification phase is provided by RF energy received from the second device. Then there is no (or only a negligible) net drain of the first device's internal battery during the proximity verification phase or communication phase. The subsequent energy-intensive operations typically are cryptographic operations, involving e.g. exponentiation or other operations that require polynomial time. In contrast, the proximity verification phase requires no arithmetic operations (to be precise: no arithmetic operations on variables at the level of the communication protocol. The low-level operation of the microprocessor may still require operations for, e.g. address calculations, but these are not considered here).

In a further preferred embodiment of the invention, the method comprises the further step of
■ given a shared secret key, either by the method as described above, or using a pre-shared key, the first and second device each picking a random nonce ("number used once");
■ the first and second device sharing, by exchanging messages over the two communication channels, their nonces, wherein the message exchange includes a measurement of the distance based on the communication signal delays;
■ the second device sending, to the first device, a command and a message authentication code (MAC) based on the command, the two nonces and the secret key known to the second device;
■ the first device verifying the integrity of the command by computing the MAC from the received command and from the two nonces and the secret key known to the first device, and comparing this MAC with the MAC received from the second device.

This allows, e.g. after a shared secret key has been established by proximity-based device pairing, to ensure that the communication partners remain located within a predetermined distance.

In a further preferred embodiment of the invention, one of the communication channels is based on RF communication, and the other one on ultrasound.

In a further preferred variant of the invention
■ the *first* device computes the distance to the second device based on communication signal delays caused by signal propagation speeds; and
■ the first device performs computationally expensive or energy intensive operations only after it has established that the distance to the second device is less than a predetermined distance.

If the distance exceeds the predetermined distance, the first device aborts communication, that is, it does not send any more messages. This allows to prevent battery draining attacks by malicious second devices.

In yet a further preferred variant of the invention
■ the *second* device computes the distance to the first device based on communication signal delays caused by the difference in signal propagation speeds; and
■ the second device aborts communication and/or generates an alert message if the distance exceeds a predetermined value.

The alert message can be detectable by a human, or transmitted or stored by technical means. This allows a malicious first device to prevent, that is further away from the second device from impersonating a first device that a user of the system thinks the second device is communicating with.

In a preferred embodiment of the invention, the step of the first device computing the distance to the second device comprises the steps of
■ triggered by an initialization signal received from the second device, the first device sending a challenge message to the second device;
■ the second device computing, from the challenge message and Further information, a response message, and sending the response message to the first device;
■ the first device computing, from the time delay between sending the challenge message and the response message, the distance to the second device;
■ the first device sending further challenge messages only if the distance does not exceed a predetermined limit.

This allows to continuously monitor the distance and to detect a spatial separation of the devices. If, in the course of communication between the two devices, the distance is exceeded, the first device preferably sends new challenge messages only after receiving another initialisation signal. This reduces energy consumption in the first device and helps to prevent malicious devices from draining the battery of the first device.

Preferably, the challenge message is a bit or a bit sequence from a nonce known only to the first device. That is, the challenge message is a number or bit sequence that is used only once, preferably a (pseudo)random sequence.

In a further preferred embodiment of the invention, the steps of sending challenge messages and receiving response messages in the first device are powered by RF energy that the first device receives from the second device. Such RF energy may be received through the initialization signal and/or the response signals. For arrangements in which the first device is powered by RF energy received through the initialisation signal, this allows the first device to force the second device to provide all the needed power, and prevents battery draining attacks.

In a further preferred embodiment of the invention, the step of controlling access of the second device to the first device, in addition to the distance, takes into account credential information. For example, the credential information is a pre-shared key known to the first and the second device, or each device stores one or more certificates that allows it to verify an electronic signature generated by another device. This allows for a further level of security, by identification.

In a preferred embodiment of the invention, the credential information is stored on a token device or storage device that is separable from the second device.

In a further preferred embodiment of the invention, the method comprises the steps of
■ the first device, being an IMD, monitoring the health condition of the implant carrier;
■ the first device, if the health condition indicates an emergency, removing the requirements for access control and allowing access without credentials and/or without proximity verification.

This allows access to an IMD in emergency situations where it would be too time-consuming or impossible to establish more secure communication with the IMD

A device according to the invention, in particular an implantable medical device, is configured to communicate with a second device, in particular with a reader for reading data from the device and optionally for controlling the device. The device comprises
■ a first transceiver for sending and receiving messages through a first communication channel;
■ a second transceiver for sending and/or receiving messages through a second communication channel;
■ wherein the first and second communication channel have different signal propagation velocities.
■ The device is configured to
   ■ exchange messages through the first communication channel and/or through the second communication channel;
   ■ to compute the distance to the second device based on communication signal delays caused by the difference in signal propagation velocities; and
   ■ depending on the computed distance, to accept data from the further device and optionally also to control access to the device.

The device can be either the first or the second device as described in the text above. In case the device is identical to a first device (such as an IMD), then the second transceiver is, for example operated only as a receiver. In case the device is identical to a second device (such as a reader), then the second transceiver is, for example operated only as a transmitter. However, the inventive device and method can also be implemented with the first device comprising, for the second communication channel, a transmitter only and the second device comprising a receiver only.

In a preferred embodiment of the inventive device, an analogue circuit for capturing and processing signals received by the second transceiver comprises countermeasures against electromagnetic influences.

Ideally, the complete analogue circuit is shielded, up to the parts after a signal captured by the second transceiver in its function as a receiver has been digitised. In particular, electric leads such as wires or electric connectors on a printed circuit board (PCB) leading to/from the second transceiver are electrically shielded and/or twisted. Preferably, the second transceiver itself, except for parts that have to be exposed in order for the second transceiver to be operated, is also shielded.

As a general principle, the principle of shielding can be applied to any distance bounding protocol and device based on a non-electromagnetic signal, such as an ultrasound signal. This means that another aspect of the invention is directed to a device implementing one side of a distance bounding protocol and using a non-electromagnetic receiver. By shielding the electromagnetically sensitive circuit parts of this non-electromagnetic receiver from electromagnetic (EM) fields, an attacker is prevented from injecting EM signals into the receiver circuit and fooling the device into perceiving them as non-electromagnetic signals. This might otherwise allow an attacking device to pretend that it is closer to the inventive device than it actually is.

Further preferred embodiments are evident from the dependent patent claims. Features of the method claims may be combined with features of the device claims and vice versa.

### BRIEF DESCRIPTION OF THE DRAWINGS

The subject matter of the invention will be explained in more detail in the following text with reference to preferred exemplary embodiments which are illustrated in the attached drawings, in which:
- Figure 1: schematically shows two devices arranged to communicate with each other; and
- Figure 2: a flow diagram of a communication method according to the invention.

The reference symbols used in the drawings, and their meanings, are listed in summary form in the list of reference symbols. In principle, identical parts are provided with the same reference symbols in the figures.

### DETAILED DESCRIPTION OF PREFERRED EMBODIMENTS

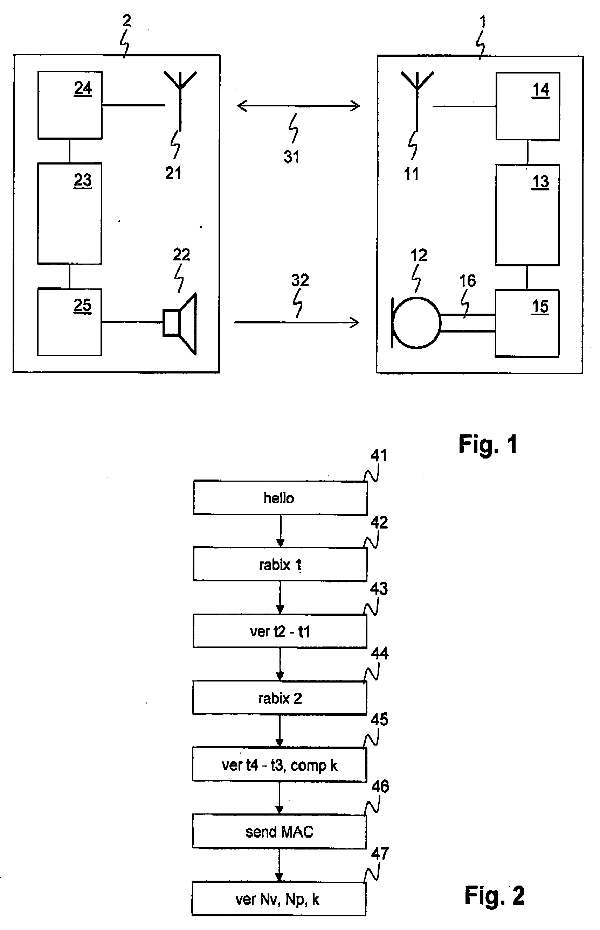

**Figure 1** schematically shows an arrangement of devices according to a preferred embodiment of the invention. A first device 1, such as an implantable medical device (IMD), henceforth called IMD 1, comprises an IMD RF (radio frequency) antenna 11 and a microphone 12 preferably sensitive to ultrasound. The IMD RF antenna 11 is functionally coupled to a IMD control unit 13 by means of a IMD antenna driver 14. The IMD antenna driver 14 digitises RF signals and provides them to the IMD control unit 13, and drives the IMD RF antenna 11 to emit signals provided by the IMD control unit 13. The IMD control unit 13 typically comprises a microprocessor for device management, communication and cryptographic operations. The microphone 12 is connected to a microphone circuit 15 by means of microphone connections 16. The microphone circuit 15 is arranged to amplify and digitise (ultra)sound signals received by the microphone 12 and provide them to the IMD control unit 13. In a preferred embodiment of the invention, the microphone circuit 15 comprises a band-pass filter to eliminate background noise and a phase-locked loop for detecting a communication frequency used by a second device.

A second device 2 or reader, henceforth called reader 2, comprises a similar structure as the IMD 1, with a reader RF antenna 21 and an ultrasound speaker 22, a reader antenna driver 24 and a speaker driver 25 operationally connected to a reader control unit 23. The ultrasound speaker 22 is driven by the speaker driver 25 based on signals provided by the reader control unit 23, the reader RF antenna 21 and reader antenna driver 24 operate as those in the IMD 1.

The two devices communicate over a first communication channel 31, in this case an RF channel, and over a second communication channel 32, in this case via ultrasound. The control units 13, 23 are programmed to interact according to the inventive method, by means of exchanging signals and messages over the first communication channel 31 and the second communication channel 32 to establish trusted and secure communication.

An access control mechanism for implantable medical devices is based on ultrasonic distance-bounding and enables an implanted medical device to grant access to its resources only to those devices that are in its close proximity. It resembles close-range communication solutions proposed in prior work in that it requires a device to be close to the IMD to get access, but differs in that it prevents the attacker from accessing the IMD from further away, regardless of the type of transceiver or antenna he has. Its security relies on the speed of the sound which can not be altered. Moreover, unlike prior proposals, our solution enables IMDs to predefine an exact range from which they can be accessed (with a high degree of accuracy). We achieve this with a new proximity-based device pairing protocol based on ultrasonic distance bounding. In this protocol, messages are cryptographically tied to the distance bounds measured by the IMD, to the device that requests access.

### System Model

Access control in this context means that a reader (potentially malicious) will try to gain access to an implantable medical device in order to readout data or send commands. The reader can be either a handheld unit or part of a bigger system but the assumption is that it is not subject to tight power and/or computational constraints. The medical device can be any device implanted into the human body, including pacemakers, implantable cardiac defibrillators (ICDs), drug delivery systems, and neurostimulators. Implantable medical devices are implanted 2-3cm below the skin.

These devices rely on wireless interfaces, allowing a doctor or medical professional to interact with the device quickly and easily, during normal consultations and in emergency scenarios. That means that a device must be accessible in the noisy and dynamic environment of a moving ambulance and at the same time prevent unauthorized access to potentially sensitive medical data.

We consider an IMD that can operate in two different modes. In a *normal mode* a reader needs to be in possession of a shared key in order to talk to the IMD and in *emergency mode* a reader just needs to be within a certain security range. In other words the emergency mode relies on proximity alone to authorize a reader.

### Attacker Model

In a first attack scenario, the attacker wants to get access to medical data stored in the implantable device or change device settings. In a second attack scenario an attacker wants to impersonate a device and make a reader talk to him. This attack might be executed by someone who wants to prevent care in an emergency situation or it could be performed by the patient himself for the purpose of insurance fraud.

The attacker can send and receive arbitrary radio and audio signals, but is subject to common computational bounds, i.e., he is not able to reverse one-way functions or solve the discrete logarithm problem. The attacker is also assumed to be outside the security range defined in the IMD (typically <10cm). In a preferred embodiment of the invention, if the malicious reader is inside the security range and the IMD is in emergency mode, the reader has free access by design.

Because the implantable medical devices run on batteries they are naturally energy constrained. That makes energy draining and DoS attacks a danger to IMDs.

### Proximity-based Access Control for Implantable Medical Devices

Access control is based on device pairing. In order for a reader to talk to an IMD it must first run a device pairing protocol and generate a shared key. This shared key is then used to gain access to the device, either to send it commands or to readout medical data. The core of the scheme is the proximity aware device pairing protocol between a hand held reader and an implanted medical device. The protocol uses ultrasonic distance bounding to determine the distance between the reader and the device. As is common practice, the terminology *prover* and *verifier* shall be used to denote the two parties throughout the rest of the application. The prover is the reader that must prove its proximity in order for data transfer to commence. The verifier is the implanted medical device that must verify the distance to the prover before accepting the connection.

### Protocol Description

The device pairing protocol is shown in Table 1 and in the flow diagram of Figure 2.

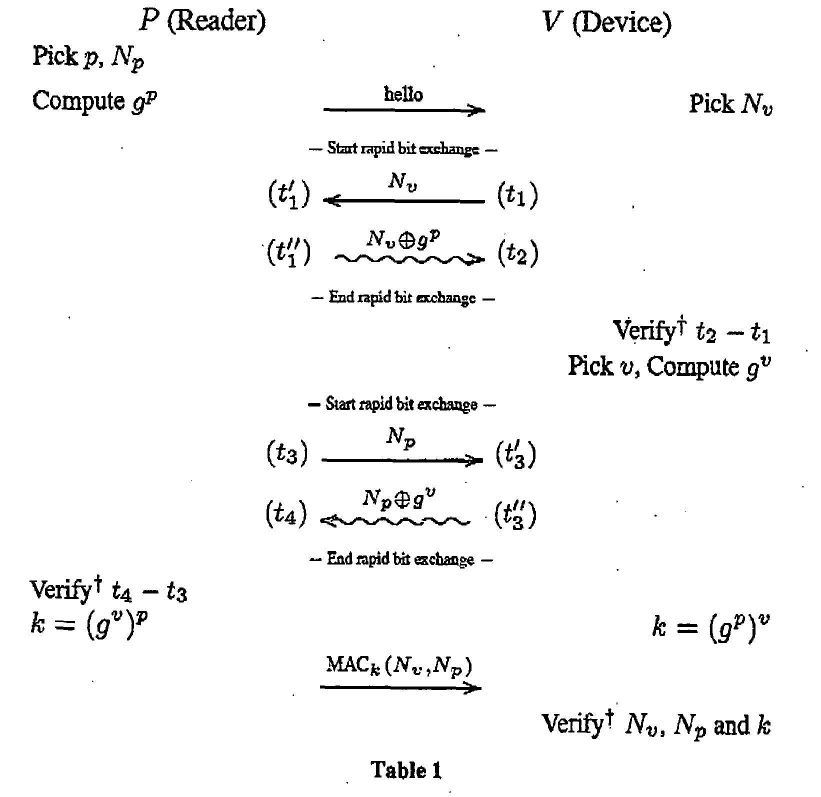

The prover will first pick a secret exponent p and a nonce *Nₚ* and then compute the public DH contribution *g^{p}*. These computations are done in advance so they will not interfere with the time-critical distance bounding steps. A 'hello' message is sent by the prover to initiate the protocol (step 41 in the flow diagram). When the verifier receives the 'hello' message it will pick a nonce *Nᵥ* and begin the rapid bit exchange phase (step 42). The verifier will send, as a challenge message, a single bit of *Nᵥ* to the prover and record the time of transmission (*t₁*) so the time-of-flight can later be calculated. The distance bounding phase must be done bit-by-bit to avoid distance shortening attacks.

The challenge message containing the first bit of *Nᵥ* is received by the reader at time *t'₁* but given that the reply must be sent via the sound channel as a response message and that the speed of sound is relatively slow compared to the propagation speed of the radio message and the delay at the prover, we consider *t*₁ = *t'*₁ = *t"*₁. The error resulting from this assumption is negligible as long as the prover replies immediately. This will be described in more detail later on.

The prover xor's the single bit message with a single bit of *g^{p}* and sends it back as a sound message. The verifier receives the sound message (response) at time *t₂*. As described above the verifier uses the time difference *t₂ - t₁* to calculate the (upper bound) distance to the prover (step 43). The distance is calculated as *d = vₛ*(*t₂ - t₁*), where *vₛ* is the speed of sound in flesh (approximately 1500m/s). If this distance is less than some predefined value, say, 5cm the protocol continues, otherwise the verifier will terminate the session. After all the bits of *Nᵥ* and *Nᵥ* ⊕ *g^{p}* have been exchanged, and passed the time-verification, the message is accepted and the DH contribution is assumed to originate from a very close reader.

After the prover has verified that the reader is within the required distance (step 43), the verifier picks *v* and computes *g^{v}*. A similar distance bounding step, i.e., a rapid bit exchange with radio challenges and response via the sound channel, is then repeated (step 44) from the verifier to the prover to ensure that the reader is talking to a device in its proximity. This is needed to prevent a (possibly far away) attacker from impersonating a device. The verification the distance, now from the reader's point of view, is based on time difference *t*₄ - *t*₃ (step 45).

Now both sides can compute the shared key as *k* = (*g^{v}*)*^{p}* and *k* = (*g^{p}*)*^{v}*, respectively. Finally, in order to let the device know that a key was successfully established, the prover sends (step 46) a final message to the verifier containing a message authentication code (MAC) of the two nonces *Nₚ* and *Nᵥ*. The MAC is, for example a keyed hash function of the two nonces, using the established key *k*. At this point, after comparing (step 47) the received MAC with the MAC it generated itself, the verifier knows that a key has been established and data transfer can continue encrypted.

### Security Analysis

Central to the device pairing protocol is the unforgeable assurance of proximity. That assurance comes from tying the DH key contributions from each party to the distance between them, by transmitting *g^{v}* and *g^{p}* over the sound channel. We assume that the attacker cannot send data on the sound channel faster than the speed of sound.

One possible attack is for the attacker to guess *Nᵥ* and then generate the sound messages in advance. If the attacker is able to generate all the sound messages and send them at the appropriate times, the attacker could pretend to be close to the verifier while actually being far away. That means that the nonce *Nᵥ* must be sufficiently random to make guessing infeasible.

The nonce *Nᵥ* is sent in the clear since it is the timing of the sound message that proves the proximity of the reader. An attacker who is further away than the allowed distance will receive the nonce at more or less the same time (the propagation time of radio signals is negligible when compared to the speed of sound) but, because he has to wait for *Nᵥ* before he can create a valid sound message, his sound message will not be able to reach the prover in time, i.e., the prover will be able to measure the distance to the attacker and conclude that he is too far away.

A similar distance bounding step is repeated in the opposite direction. This proves to then reader that the IMD is also within the specified distance, eliminating impersonation attacks. Since the two DH contributions are sent over the sound channel they are directly linked to the distance between the reader and IMD, which also makes the key *k*=*g^{vp}* directly linked to the distance as well.

In order to limit the effectiveness of battery draining attacks the IMD only generates its public DH contribution - which is an computationally expensive and thus also power consuming operation - *after* the distance to the reader has been verified. That way only the initial nonce must be generated at the start of each session.

The final message from the prover to the verifier confirms the key. After executing this protocol the verifier knows that a valid key has been generated with a prover and that this prover is within the allowed distance. At this point the verifier can start transmitting data using the generated key *k* or send another message to the prover confirming the key.

### Side Channel Attack Protection

One of the most important assumptions in the security analysis is that the attacker cannot send data on the sound channel with a signal that propagates faster than the speed of sound.

While this assumption sounds perfectly reasonable there are pitfalls that an attacker might utilize. It was discovered that it is possible to send a radio signal to the IMD that will induce a current in the audio receiver circuit just as if the IMD received a sound signal. This could happen, for example, if there are two small wires going from the reception circuit to a piezo element (working as a microphone). This would be enough to pick up a radio signal of about the same order of magnitude as the audio transmission. The countermeasure to this is effective RF shielding of, ideally, all analogue parts of the reception circuit, and in particular of connecting leads. If proper shielding is not in place, a strong attacker can effectively send an 'audio' transmission at the speed of light!

### Propagation Time and Processing Delay

The propagation time of the radio signal and the delay at the prover is negligible, relative to the propagation time of the sound signal. That its, *t*₁ *= t'*₁ = *t"*₁ for practical purposes. Furthermore, the speed of sound is higher when the sound propagates through the human body than when the sound propagates through air. The speed of sound through the human body is approximately 1500m/s which is about three times the speed through air. Assuming a speed of sound of 1500m/s when defining the maximum distance from which the device can be accessed, it follows that any distance the signal has to travel through air to get to the reader will be counted three times because the signal travels three times slower. That means that any additional distance to an attacker outside the allowed access radius is amplified thus making it even harder to cheat the system.

### Protocol Extensions

### Combining Proximity and Credential-Based Solutions

It is likely that patients will be provided some form of credential (a smart card, USB stick or password) that shares a secret with the implanted medical device. This credential would be used by a reader (operated by the doctor) to actually get access the IMD when necessary. However the credential-based approach has several drawbacks, since it can be stolen, or a doctor can be fooled by a nearby IMD, e.g. for insurance fraud purposes. If the patient does not carry his credential, no one can access the IMD even in case of emergency. The inventive scheme can complement the credential-based solutions to solve these issues. In a *normal mode of operation,* the patient carries the credential token and provides it to the doctor that needs to access the IMD. In an *emergency mode of operation,* the doctor does not have access to the credential token.

### Normal Mode of Operation

The patient carries an authorization credential token (USB token, smart card, password, etc.) that shares a secret key *k_{shared}* with the IMD. When a doctor needs to access the IMD, he gets the credential from the patient and provides it to the reader. The same proximity aware device pairing protocol shown in Table 1 is run between the reader and the IMD except that, in addition the shared key *k_{shared}* is included in the MAC in the final message. Once the protocol has been executed, each party has the assurance that the other party is within its security range and has derived a key *k* that is used to secure their future communication.

By verifying that the IMD is in the proximity of the reader, the doctor has the assurance that his reader is communicating with the patient's IMD.

Note that since, in this mode of operation, the IMD and the reader share a secret, in an alternative preferred embodiment of the invention, the Diffie-Hellman key exchange could easily be avoided if necessary. In fact, both parties could derive a key *k* from the shared secret *k_{shared}* and the exchanged nonces. However, the ephemeral Diffie-Hellman key exchange protocol provides forward security, which can be a valuable property.

### Emergency Mode of Operation

In this mode of operation, it is assumed that the authorization token is not available. With most existing systems, in this situation, wireless communication is not possible unless the IMD is activated by a magnetic read switch. Again, the protocol shown in Table 1 can be used: With this solution, both the reader and the IMD verify that they are within each other's security range and generate a temporary secret key. An attacker won't be able to get access to the victim's IMD from a remote location, however, he could potentially establish a key with the IMD if he gets close to the patient, without having to steal his credential. In a further preferred embodiment of the invention, in the Emergency mode of operation, the security range should is much smaller than in the normal mode of operation, for example, the range of less than 10 cm, and preferably less than 4 or 2 cm. This would require the attacker to almost have physical contact with his victim.

In a further preferred embodiment of the invention, input data from other sensors are used to reinforce the security of the emergency mode of operation. For example, if the IMD is equipped with an accelerometer, the IMD is configured to verify that the reader is close, as described above, but also that the patient is lying down. Furthermore, in another preferred embodiment of the invention, if the IMD detects an emergency situation (stroke, heart failure, etc.), access control is deactivated all together.

### Proximity-Based Commands

In the above, the proximity-based scheme has been described for securing the IMD - reader pairing during the normal and emergency modes of operation. However, this approach can be extended to any other aspect of IMD--reader communication.

A doctor might want to access an IMD for several reasons. One reason could be to remotely monitor a patient and retrieve logging/history data. Another reason could be to modify the parameters of the IMD or reconfigure the device. The second type of operation is clearly more critical and requires stronger security, since it can potentially threaten the life of the patient. The first type would only violate privacy if performed by a non-authorized user. It is therefore reasonable to apply different security policies for each of these operations. Therefore, in a further preferred embodiment of the invention, as long as the implanted medical device is in the normal mode of operation, critical commands such as remote reconfiguration or parameter setting are only processed if issued by a reader that is in its proximity, closer than a first distance limit, such as 2, 4 or 5 cm. Remote monitoring of the IMD via a secured channel is however allowed if the reader is closer than a second distance limit, such as 8 or 10 or 15 cm.

In order to verify the proximity of the reader when it sends a command, a command proximity verification protocol is implemented, as illustrated in the following table. It is assumed that the reader and the IMD share a secret key, *k*, i.e., that both devices have been securely paired already.

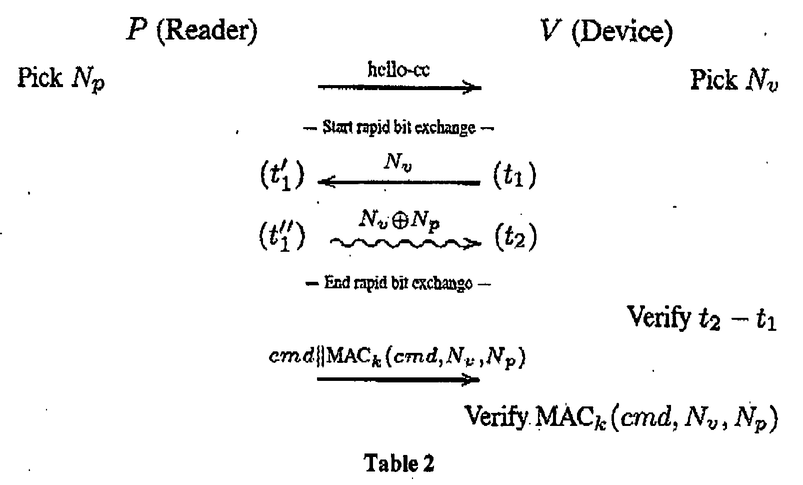

When a reader wants to send a critical command to an IMD, it starts by sending a 'hello-cc' to initiate the protocol. The IMD picks a nonce *Nᵥ* and replies with the first bit of *Nᵥ*. The IMD also starts a timer so the time-of-flight of the sound message can be measured. The reader responds immediately with a single bit of its own nonce xor'ed with *Nᵥ* and this continues until there are no more bits in the nonces, or until the IMD aborts the protocol because the estimated distance is outside the security range.

Once the distance bounding phase of the protocol is over the reader sends the command *cmd* along with a MAC of the command and the nonces. If the IMD is able to verify the MAC it knows that *cmd* came from within the security distance and will process the command.

Although it is assumed in the proximity-based command protocol that the two devices share a secret key, this protocol could still be useful in scenarios where the only policy for being able to issue command is to be close the device. The modification to the protocol would then be to replace the MAC function with a regular hash function. The security would, of course, be lower but could still be acceptable for some applications.

### Robustness

Because robustness is a criterion, in further preferred embodiment of the invention, the proximity aware device pairing protocol of Table 1 (or Table 2) is allowed to continue, despite transmission errors on the sound channel. This is an optional addition to the protocol and enables device pairing in extremely loud environments at the cost of some security. After the rapid bit exchange phase, the prover (or verifier) sends a radio message containing the exact same data (*Nᵥ* ⊕ *Nₚ*) as was sent in the sound messages. Doing that will enable the verifier (or prover) to use the arrival time of the sound messages to detect proximity but since the same data was transmitted via the radio channel (which presumably is immune to audio noise) it doesn't matter if part of the audio message is wrong. It should be emphasized that this extra radio message is sent after the distance bounding phase has completed successfully. In order for an attacker to abuse this protocol, he must already have cheated the distance bounding phase, i.e., sent all replies at the correct times, otherwise the protocol would have been aborted.

If the verifier (or prover) is willing to accept some transmission errors in the audio messages, it reduces the guessing space for the attacker. However, as long as enough bits are correct, the verifier (or prover) can be fairly certain that the audio messages where not guessed in advance and sent by an attacker. Depending on the number of bits transmitted, the verifier requires a corresponding number of bits, e.g. 75%, to be correct, in order to establish the proximity pairing.

While the invention has been described in present preferred embodiments of the invention, it is distinctly understood that the invention is not limited thereto, but may be otherwise variously embodied and practised within the scope of the claims.

### LIST OF DESIGNATIONS

- 1: first device, implantable medical device
- 11: IMD RF antenna
- 12: microphone
- 13: IMD control unit
- 14: IMD antenna driver
- 15: microphone circuit
- 16: microphone connection
- 2: second device, reader
- 21: reader RF antenna
- 22: ultrasound speaker
- 23: reader control unit
- 24: reader antenna driver
- 25: speaker driver
- 31: first communication channel, radio frequency
- 32: second communication channel, ultrasound

## Claims

1. A method for communicating between a first device (1), in particular an implantable medical device, and a second device (2), in particular a reader for reading data from the first device and optionally for controlling the first device (1), the method comprising the steps of
■ the first and second device (1, 2) communicating by exchanging messages that are based on signals that are (1) transmitted through a first communication channel (31) and/or through a second communication channel (32), wherein the first and second communication channel (31, 32) have different signal propagation velocities;
■ at least one of the first and second device (1, 2) computing the distance to the other device (2, 1) based on communication signal delays caused by signal propagation velocities;
**characterised in that** the method comprises the further steps of
■ controlling access of the second device (2) to the first device (1) depending on the computed distance.

2. The method of claim 1, comprising the further step of
■ the first and second device (1, 2), by exchanging the messages, establish a shared secret key.

3. The method of claim 1 or claim 2, comprising the further step of
■ given a shared secret key, the first and second device (1, 2) each picking a random nonce;
■ the first and second device (1, 2) sharing, by exchanging messages over the two communication channels (31, 32), their nonces, wherein the message exchange includes a measurement of the distance based on the communication signal delays;
■ the second device (2) sending, to the first device, a command and a message authentication code (MAC) based on the command, the two nonces and the secret key known to the second device (2);
■ the first device (1) verifying the integrity of the command by computing the MAC from the received command and from the two nonces and the secret key known to the first device (1), and comparing this MAC with the MAC received from the second device (2).

4. The method of one of the preceding claims, wherein the first device is an implantable medical device (IMD) and the second device is a reader device for reading data from the IMD and optionally also setting parameters in the IMD or otherwise controlling the IMD.

5. The method of one of the preceding claims, wherein one of the communication channels (31, 32) is based on RF communication, and the other one on ultrasound.

6. The method of one of the preceding claims, comprising the further step of
■ the *first* device (1) computing the distance to the second device (2) based on communication signal delays caused by the difference in signal propagation speeds; and
■ the first device (1) performing energy-intensive operations only after it has established that the distance to the second device (2) is less than a predetermined distance.

7. The method of one of the preceding claims, the further step of
■ the *second* device (2) computing the distance to the first device (1) based on communication signal delays caused by the difference in signal propagation speeds; and
■ the second device (2) aborting communication and/or generating an alert message if the distance exceeds a predetermined value.

8. The method of one of the preceding claims, wherein the step of the first device (1) computing the distance to the second device (2) comprises the steps of
■ triggered by an initialization signal received from the second device (2), the first device (1) sending a challenge message to the second device;
■ the second device (2) computing, from the challenge message and further information, a response message, and sending the response message to the first device (1);
■ the first device (1) computing, from the time delay between sending the challenge message and the response message, the distance to the second device (2);
■ the first device (1) sending further challenge messages only if the distance does not exceed a predetermined limit

9. The method of claim 8, wherein the challenge message is a bit or a bit sequence from a nonce known only to the first device (1).

10. The method of claim 8 or 9, wherein the steps of sending challenge messages and receiving response messages in the first device (1) are powered by RF energy that the first device (1) receives from the second device (2).

11. The method of one of the preceding claims, wherein the step of controlling access of the second device (2) to the first device (1), in addition to the distance, takes into account credential information

12. The method of claim 11, wherein the credential information is a pre-shared key known to the first and the second device (1, 2), or the credential information is a cryptographic certificate, and preferably the credential information is stored on a storage device that is separable from the second (2) device.

13. The method of one of the preceding claims, comprising the further steps of
■ the first device (1), being an IMD, monitoring a health condition of an implant carrier;
■ the first device (1), if the health condition indicates an emergency, removing the requirements for access control and allowing access without credentials and/or without proximity verification.

14. The method of one of the preceding claims, wherein the first device (1) comprises two or more levels of access, and the method comprises the further step of
■ the first device (1) controlling access to the different levels of access depending on the value of the computed distance.

15. A device (1; 2), in particular an implantable medical device, configured to communicate with a further device (2; 1), in particular with a reader for reading data from the device (1; 2) and optionally for controlling the device (1; 2), the device (1; 2) comprising
■ a first transceiver (11; 21) for sending and receiving messages through a first communication channel (31);
■ a second transceiver (12; 22) for sending and/or receiving messages through a second communication channel (32);
■ wherein the first and second communication channel (31, 32) have different signal propagation velocities;
■ the device (1; 2) being configured to
o exchange messages through the first communication channel (31) and/or through the second communication channel (32);
o to compute the distance to the further device (2; 1) based on communication signal delays caused by the difference in signal propagation velocities; and
o depending on the computed distance, to accept data from the further device (2; 1) and optionally also to control access to the device (1; 2).

16. The device (1) of claim 15, wherein an analogue circuit (15) for capturing and processing signals received by the second transceiver (12) comprises countermeasures against electromagnetic influences.
